# EUROPEAN PATENT SPECIFICATION

(11) **EP 0 333 201 B1**
(45) Date of publication and mention of the grant of the patent: **28.09.1994**
(21) Application number: 89104763.1
(22) Date of filing: 17.03.1989
(51) Int. Cl.: A61K 37/02, C12P 21/02, C12N 15/00, G01N 33/68

(54) **Human heat shock factor**
Menschlicher Hitzeschock-Faktor
Facteur du choc thermique humain

(30) Priority: 18.03.1988 US 169965; 25.01.1989 US 301417
(43) Date of publication of application: 20.09.1989
(73) Proprietor: THE GENERAL HOSPITAL CORPORATION, Boston, MA 02114 (US)
(72) Inventor: Kingston, Robert E., Brighton, MA 02135 (US); Schuetz, Thomas J., Jamaica Plain, MA 02130 (US)
(74) Representative: Fischer, Hans-Jürgen, Dr.

(56) References cited:
- ANN. REV. BIOCHEM., vol. 55, 1986, pages 1151-1191; S. LINDQUIST: "The heat-shock response"
- MOLECULAR AND CELLULAR BIOLOGY, vol. 7, no. 4, April 1987, pages 1530-1534,American Society for Microbiology; R.E. KINGSTON et al.: "Heat-inducible humanfactor that binds to a human hsp70 promoter"
- NATURE, vol. 309, 17th May 1984, pages 229-234; C. WU: "Two protein-binding sites in chromatin implicated in the activation of heat-shock genes"
- PROC. NATL. ACAD. SCI., vol. 83, February 1986, pages 629-633; B.J. WU et al.:"Human HSP70 promoter contains at least two distinct regulatory domains"
- EXPERIMENTAL CELL RESEARCH, vol. 165, 1986, pages 1-10, Academic Press Inc.; K.W. LANKS: "Modulators of the eukaryotic heat shock response"
- THE EMBO JOURNAL, vol. 6, no. 10, 1987, pages 3035-3041, IRL Press Ltd, Oxford,GB; P.K. SORGER et al.: "Purification and characterization of a heat-shockelement binding protein from yeast"
- ANALYTICAL BIOCHEMISTRY, vol. 166, 1987, pages 158-171, Academic Press Inc.; E.WESTMAN et al.: "Separation of DNA restriction fragments by ion-exchange chromatography on FPLC columns mono P and mono Q"
- NUCLEIC ACIDS RESEARCH, vol. 8, no. 16, 1980, pages 3721-3728, IRL Press Ltd,London, GB; G. HERRICK: "Site-specific DNA-affinity chromatography of the lacrepressor"
- NUCLEIC ACIDS RESEARCH, vol. 5, no. 3, March 1978, pages 1059-1074, Information Retrieval Ltd.,London GB; H. BÜNEMANN et al.: "Base specific fractionation of double stranded DNA: affinity chromatography on a novel type of adsorbant"
- J. CHEM. TECH. BIOTECHNOL. vol. 36, 1986, pages 345-350; D.K.R. LOW: "The use of the F.p.l.c.R system in method development and process monitoring for industrial protein chromatography"

## Description

This application is a continuation-in-part of U.S. Patent Application Serial No. 169,965 filed March 18, 1988.

The present invention relates to a human heat shock factor, and this factor for the diagnosis and treatment of disease and stress. The invention also pertains to the production of this factor by recombinant DNA techniques, and to DNA sequences which encode this factor.

Cells exposed to elevated temperatures or certain chemical agents respond to such stress by inducing the synthesis of a small set of proteins. This cellular response is termed a "heat shock response." The proteins synthesized in response to stress are termed "heat shock proteins" or "HSP." The heat shock response is believed to be ubiquitous, and has been observed in prokaryotic as well as eukaryotic cells and organisms (including man). The synthesis of the heat shock proteins is highly significant since failure to produce these proteins in response to stress is associated with cell death and with tissue and organ injury. The heat shock proteins are believed to play a role in attenuating the severity of the stress, and, in thereby returning the effected cell to a quiescent state. Excellent reviews of the heat shock phenomenon are provided by Lanks, K.W. (Exper. Cell Res. 165:1-10 (1986)) and Lindquist, S. (Ann. Rev. Biochem. 55:1151-1191 (1986)).

The heat shock proteins have been found to possess functions which are unrelated to, and independent of, their role in the heat shock phenomenon. The genes which encode the heat shock proteins (i.e. hsp genes) have been found to be differentially expressed during embryonic development (Bensaude, O. et al., Nature 305:331 (1983)), during differentiation of specific cell types such as myoblasts, embryonal carcinoma, and erythroid cells (Atkinson, B.G., J. Cell. Biol. 89:666 (1986); Atkinson, B.G. et al., Can. J. Biochem. Cell. Biol. 61:404 (1983) ; Morange, M. et al., Molec. Cell. Biol. 4:730 (1984); Singh, M.K. et al., Nature 309:631 (1984)). Neoplastic transformation is also associated with a change in the level of the heat shock proteins (Lanks, K.W., Exper. Cell Res. 165:1-10 (1986)).

The genes for the heat shock proteins have been found to possess highly conserved 5′ sequences which contain a rotationally symmetric consensus sequence known as the "Heat Shock Element" or "HSE". A heat shock transcriptional factor ("HSTF" or "HSF") has been identified which is believed to bind to the HSE sequences, and to play a role in the transcription of the genes which encode the heat shock proteins (Lindquist, S. (Ann. Rev. Biochem. 55:1151-1191 (1986); Parker, C.S. et al., Cell 36:357-369 (1984); Parker, C.S. et al., Cell 37:253-262 (1984); Wu, C., Nature 286:854-860 (1980); Wu, C., Nature 309:229-241 (1984); Wu, C., Nature 317:84-87 (1985)). As such, this factor is capable of regulating and augmenting the heat shock response of an individual.

Thus, this factor, and agents which promote the activity of this factor, provide a therapy for diseases which are associated with the heat shock response. Because this factor has not been sufficiently characterized to permit its use in the therapy for such diseases, or the permit the identification of agents which promote its activity, it has not previously been possible to employ HSF (or such agents) as the basis for a treatment of such diseases. Thus, a need exists for a means for purifying and producing HSF and its derivatives, antagonists, and agonists.

The present invention relates to Heat Shock Factor, and its derivatives, antagonists, and agonists. The invention also pertains to the production and purification of such compounds from natural and recombinant sources. The invention additionally pertains to diagnostic and therapeutic applications for such compounds.

In detail, the invention concerns a compound comprising Heat Shock Factor substantially free of natural contaminants, or derivatives thereof.

The invention further includes a therapeutic medicament comprising Heat Shock Factor or an agent which activates Heat Shock Factor.

The invention also includes a recombinant nucleic acid molecule comprising a sequence which encodes Heat Shock Factor.

The invention also pertains to the above compound, wherein the compound is prepared by a process comprising DNA affinity chromatography.

The invention also pertains to a method for purifying Heat Shock Factor which comprises subjecting a sample suspected of containing the factor to DNA affinity chromatography.

The invention additionally pertains to a method for diagnosing the stress condition of a patient which comprises assaying for the presence of activated heat shock factor.

### The Heat Shock Response

The present invention derives, in part, from studies of the response of human cells to changes in the external environment. Human cells respond to a variety of environmental changes by coordinately inducing an appropriate set of genes, frequently via the sequence-specific binding of a transcription factor to regulatory promoter sites (Serfling E. et al., Trends in Genet. 1:224 (1985); McKnight, S. et al., Cell 46:795 (1986); Maniatis, T. et al., Science 236:1237 (1987)). In several instances, coordinate regulation of mammalian genes appears to involve modification of a pre-existing transcription factor to a form that can bind the regulatory element of a promoter (Yamamoto, K.Ann. Rev. Genet. 19:209 (1985); Sen, R. et al., Cell 47:921 (1986); Prywes, R. et al., Cell 47:777 (1986); Hayes, T.E. et al., Proc. Natl. Acad. Sci. USA 84:1272 (1987); Kingston R.E. et al., Mol. Cell. Biol. 7:1530 (1987); Zimarino, V. et al., Nature 327:727 (1987). Little is known about the biochemical changes that cause this alternation of DNA-binding capability. These changes are clearly an essential link in the transduction of an environmental signal to an appropriate response: induction of a specific set of genes. Determining the mechanism by which transcription factor binding can be induced is therefore essential to understanding many regulatory pathways.

Environmental signals lead to post-translational changes in the DNA-binding ability of several factors that regulate mammalian transcription. In prokaryotic systems, environmental signals can lead to phosphorylation of DNA-binding regulatory proteins: for example, the ntrB product phosphorylates the NtrC protein in response to nitrogen limitation, resulting in activation of NtrC. Similarly, yeast HSF becomes phosphorylated in response to heat.

As discussed above, the heat shock response, which occurs in organisms from bacteria to humans involves the enhanced synthesis of a set of protective proteins encoded by the hsp genes. The transcription of these genes is regulated by "heat shock factor" ("HSF"). HSF is normally produced at a low level, even in quiescent cells. In response to stress, however, the HSF concentration in a cell increases, and is converted into an activated form. The activated form of HSF is capable of binding to the HSE sequences, and of thereby mediating the enhancement of the transcription of the heat shock protein-encoding genes. Thus, the heat shock response is an example of coordinated gene regulation.

When human cells are heated to 43°C, transcription of the heat shock genes has been found to be induced (Ritossa, F.M., Experientia (Basel) 18:571 (1962); Nover, L., Heat Shock of Eukaryotic Cells. Springer, Berlin (1984); Craig, E.A., CRC Cit. Rev. Biochem. 18:239 (1985); Pelham, H.R.B., Trends Genet. 1:31 (1985); Lindquist, S., Ann. Rev. Biochem. 55:1151 (1986)) through the palindromic heat shock element s(HSEs) located upstream of each promoter (Pelham, H.R.B. et al., EMBO J., 1:1473 (1982); Mirault, M.E. et al., EMBO J. 1:1279 1:1279 (1982): Wu, B. et al., Mol. Cell. Biol.5:330 (1985); Voellmy et al., Proc. Natl. Acad. Sci. USA 82:4949 (1985); Drabent B. et al., Nucl. Acids Res. 14:8933 (1986); Berger, E.M. et al., Somat. Cell. Molec. Genet. 12:433 (1986); Wu. B.J. et al., Proc. Natl. Acad. Sci. USA 83:629 (1986)).

Binding of HSF to the HSE has been found to directly stimulate transcription in Drosophila, and is believed to similarly stimulate expression of heat shock genes in other organisms (Kingston R.E. et al., Mol. Cell. Biol. 7:1530 (1987); Wu, C., Nature 309:229 (1984); Parker, C.S. et al., Cell, 37:273 (1984); Morgan, W.D. et al., Mol. Cell. Biol. 7:1129 (1987); Wiederrecht, G.et al., Mol. Cell. Biol. 7:1129 (1987); Wiederrecht, G. et al., Cell 48:507 (1987); Wu et al., Science 238:1249 (1987); Sorger, P.K. et al., Nature 329:81 (1987)). The ability of HSF to bind the HSE is heat-inducible, by a post-translational mechanism, in Drosophila and human cells (Kingston R.E. et al., Mol. Cell. Biol. 7:1530 (1987); Zimarino, V. et al., Nature 327:727 (1987); Sorger P.K. et al., Nature 329:81 (1987)).

Induction of the ability of a factor to bind to the HSE of heat shock genes is apparently central to control of the heat shock response in Drosophila and human cells. The induction of binding ability occurs as a direct response to temperature, as binding is induced in the cytoplasmic extract with approximately the same temperature profile as in an intact cell. This change in binding ability is not the only alteration of HSF that occurs when cells are heated. HSF from heated cells is apparently phosphorylated to a significantly greater degree than HSF activated in vitro. HSF is activated in human cells by at least two steps: an induction of binding ability followed by phosphorylation. Heating a cytoplasmic extract recreates only the first of these steps, resulting in a factor with lower apparent molecular weight. This putative phosphorylation event may increase the ability of HSF to activate transcription, or may alter some other characteristic critical to HSF function.

Thus, in summary, HSF is a transcriptional factor which, when activated by a cell's exposure to heat or stress, mediates the enhanced transcription of the hsp genes, and thereby causes an increase in the cellular concentration of heat shock proteins. The heat shock proteins act to attenuate the harmful effects of the stress.

### The Purification of HSF

Activated HSF is preferably purified from HeLa cells, however, other human cells may be employed for this purpose. The cells are cultured in a suitable culture medium, preferably Minimal Essential Medium (Joklik's Modification) supplemented with serum. After the cells have reached a desirable density, they are harvested and used to prepare nuclear extracts. Such extracts are preferably prepared according to the method of Dignam, D. et al., (Nucl. Acids Res 11:1475 (1983)).

The nuclear extract is then passed through a double stranded DNA-cellulose column. Bound material is eluted from the column using a step elution with 0.3 M KCl. HSF activity is preferably assayed using the gel electrophoresis assay of Kingston, R.E. et al. (Molec. Cell. Biol. 7:1530 (1987) which reference is herein incorporated by reference). Fractions containing HSF activity are pooled, in preparation for further purification.

A double-stranded DNA affinity column is prepared which preferably uses a concatemer of a self-complementary, HSE-containing, 28 base oligonucleotide having the sequence:
5′ - GATCCTAGAAGCTTCTAGAAGCTTCTAG - 3′
Such a column is preferably prepared in accordance with the method of Kadonaga et al. (Proc. Natl. Acad. Sci. USA 83:5889 (1986). The oligonucleotide is preferably covalently attached to Sepharose CL-2B (Pharmacia) using cyanogen bromide to form the affinity column.

The pooled HSF-containing fractions are then applied to the above-described sequence double-stranded DNA affinity column. Activated HSF binds to the column, and can be eluted from the column in a 0.75 M KCl salt wash. Fractions exhibiting HSF activity are then pooled in preparation for further purification.

The pooled fractions are then preferably diluted to 0.1 M KCl and applied to a MONO Q FPLC column (Pharmacia). The bound material is preferably eluted with a linear gradient of KCl (0.1 to 1.0 M). HSF activity is assayed, preferably in the manner described above, and active fractions are pooled. HSF activity is eluted from the column at 0.3-0.6 M KCl.

Using the above-described procedure, activated HSF, capable of binding to DNA can be isolated and purified.

### HSF and its Functional Derivatives, Agonists and Antagonists

The present invention pertains to activated heat shock factor ("HSF"), to therapeutic fragments of this factor, as well as to functional derivatives, agonists and antagonists of this factor. The invention especially concerns agents which are capable of activating HSF into is active form.

A "functional derivative" of HSF is a compound which possesses a biological activity (either functional or structural) that is substantially similar to a biological activity of HSF. The term "functional derivatives" is intended to include the "fragments," "variants," "analogues," or "chemical derivatives" of a molecule. A "fragment" of a molecule such as HSF, is meant to refer to any polypeptide subset of the molecule. A "variant" of a molecule such as HSF is meant to refer to a molecule substantially similar in structure and function to either the entire molecule, or to a fragment thereof. A molecule is said to be "substantially similar" to another molecule if both molecules have substantially similar structures or if both molecules possess a similar biological activity. Thus, provided that two molecules possess a similar activity, they are considered variants as that term is used herein even if the structure of one of the molecules not found in the other, or if the sequence of amino acid residues is not identical. An "analogue" of a molecule such as HSF is meant to refer to a molecule substantially similar in function but not in structure to either the entire molecule or to a fragment thereof. As used herein, a molecule is said to be a "chemical derivative" of another molecule when it contains additional chemical moieties not normally a part of the molecule. Such moieties may improve the molecule's solubility, absorption, biological half life, etc. The moieties may alternatively decrease the toxicity of the molecule, eliminate or attenuate any undesirable side effect of the molecule, etc. Moieties capable of mediating such effects are disclosed in Remington's Pharmaceutical Sciences (1980). Procedures for coupling such moieties to a molecule are well known in the art.

An "antagonist" of HSF is a compound which exhibits the function of HSF. Such antagonists can be immunoglobulins (such as, for example, monoclonal or polyclonal antibody, of active fragments of such antibody).

Polyclonal antibody capable of binding to activated HSF can be prepared by immunizing a mammal with a preparation of activated HSF or functional derivative of HSF. Methods for accomplishing such immunizations are well known in the art. Monoclonal antibodies (or fragments thereof) can also be employed to assay for the presence (or amount) of activated HSF in a particular biological sample. Such antibodies can be produced by immunizing splenocytes with activated HSF (by modifying the procedures of Kohler et al. (Nature 256:495 (1975): Eur. J. Immunol. 6:511 (1976); Euro J. Immunol. 6:292 (1976)).

In addition to the above methods, antibodies capable of binding to activated HSF may be produced in a two step procedure through the use of anti-idiotypic antibodies. Such a method makes use of the fact that antibodies are themselves antigens, and that, therefore, it is possible to obtain an antibody which binds to a second antibody. In accordance with this method, antibodies capable of binding to activated HSF are used to immunize an animal. The splenocytes of such an animal are then used to produce hybridoma cells, the and hybridoma cells are screened to identify clones which produce antibody whose ability to bind to anti-activated HSF antibodies can be specifically blocked by activated HSF protein. Such antibodies comprised anti-idiotypic antibodies to the anti-HSF antibody. Such antibodies can be used to immunize an animal, and thereby induce the formation of antibodies capable of binding to activated HSF.

In addition to providing additional HSF (or a functional derivative of HSF) to a subject, the efficacy of HSF in a subject can be increased by the administration of an agonist of HSF to a subject. The invention additionally pertains to such agonists of HSF. An agonist of HSF is any compound which is capable of increasing the efficacy of a function of HSF. Examples of such agonists include an agent which promotes the synthesis of HSF by the subject, an agent which promotes the activation of pre-existing HSF, etc. Agents which promote the activation of pre-existing HSF are the preferred agonists and therapeutic agents of the present invention.

HSF or agents which promote the activation of HSF may be obtained either synthetically, through the use of recombinant DNA technology, or by proteolysis. The therapeutic advantages of such agents may be augmented through the combined administration of several agents. The scope of the present invention is further intended to include functional derivatives of HSF which lack one, two or more amino acid residues, or which contain altered amino acid residues, so long as such derivatives exhibit the capacity to influence the heat shock response.

The compounds of the present invention are said to be "substantially free of natural contaminants" if preparations which contain them are substantially free of materials with which these products are normally and naturally found.

### Methods for Preparing HSF

The heat shock factor of the present invention may be obtained by natural processes (such as, for example, by inducing the production of HSF from a human or animal cell); by synthetic methods (such as, for example, by using the Merrifield method for synthesizing polypeptides to synthesize HSF, functional derivatives of HSF, or agonists or antagonists of HSF (either immunoglobulin or non-immunoglobulin)); or by the application of recombinant technology (such as, for example, to produce the HSF of the present invention in diverse hosts (i.e., yeast, bacteria, fungi, cultured mammalian cells, etc.), or from recombinant plasmids or viral vectors). The choice of which method to employ will depend upon factors such as convenience, desired yield, etc. It is not necessary to employ only one of the above-described methods, processes, or technologies to produce HSF; the above-described processes, methods, and technologies may be combined in order to obtain HSF. It is most preferable to prepare HSF by cloning and expressing a gene or cDNA sequence which encodes the preactivated HSF protein. Such gene cDNA sequence in hereinafter termed the "HSF gene" or "HSF cDNA sequence."

Any of a variety of procedures may be used to clone either the HSF gene (or, equivalently, a cDNA sequence which encodes HSF). One such method entails analyzing a shuttle vector library of cDNA inserts (derived from an HSF expressing cell) for the presence of an insert which contains the HSF gene or cDNA sequence. Such an analysis may be conducted by transfecting cells with the vector and then assaying for HSF expression.

The preferred method for cloning this gene entails determining the amino acid sequence of the HSF molecule. To accomplish this task, HSF protein may be purified, as described above, and analyzed by automated sequenators. Alternatively, the molecule may be fragmented as with cyanogen bromide, or with proteases such as papain, chymotrypsin or, preferably, trypsin (Oike, Y. et al., J. Biol. Chem. 257:9751-9758 (1982); Liu, C. et al., Int. J. Pept. Protein Res. 21:209-215 (1983)). Although it is possible to determine the entire amino acid sequence of HSF, it is preferable to determine the sequence of peptide fragments of the molecule. If the peptides are greater than 10 amino acids long, the sequence information is generally sufficient to permit one to clone a gene such as the gene for HSF (or a cDNA gene sequence of the HSF gene).

Once one or more suitable peptide fragments have been sequenced, the DNA sequences capable of encoding them are examined. Because the genetic code is degenerate, more than one codon may be used to encode a particular amino acid (Watson, J.D., In: Molecular Biology of the Gene, 3rd Ed., W.A. Benjamin, Inc., Menlo Park, CA (1977), pp. 356-357). The peptide fragments are analyzed to identify sequences of amino acids which may be encoded by oligonucleotides having the lowest degree of degeneracy. This is preferably accomplished by identifying sequences that contain amino acids which are encoded by only a single codon. Although occasionally such amino acid sequences may be encoded by only a single oligonucleotide, frequently the amino acid sequence can be encoded by any of a set of similar oligonucleotides. Importantly, whereas all of the members of the set contain oligonucleotides which are capable of encoding the peptide fragment and, thus, potentially contain the same nucleotide sequence as the gene sequence which encodes the peptide fragment, only one member of the set contains a nucleotide sequence that is identical to the nucleotide sequence of this gene sequence. Because this member is present within the set, and is capable of hybridizing to DNA even in the presence of the other members of the set, it is possible to employ the unfractionated set of oligonucleotides in the same manner in which one would employ a single oligonucleotide to clone the gene (or cDNA sequence) that encodes the peptide.

In a manner exactly analogous to that described above, one may employ an oligonucleotide (or set of oligonucleotides) which have a nucleotide sequence that is complementary to the oligonucleotide sequence or set of sequences that is capable of encoding the peptide fragment.

A suitable oligonucleotide, or set of oligonucleotides which is capable of encoding a fragment of the HSF gene (or which is complementary to such an oligonucleotide, or set of oligonucleotides) is identified (using the above-described procedure), synthesized, and hybridized, by means well known in the art, against a DNA or, more preferably, a cDNA preparation derived from human cells which are capable of expressing HSF gene sequences. Techniques of nucleic acid hybridization are disclosed by Maniatis, T. et al., In: Molecular Cloning, a Laboratory Manual, Cold Spring Harbor, NY (1982), and by Haymes, B.D. et al., In: Nucleic Acid Hybridization, a Practical Approach, IRL Press, Washington, DC (1985), which references are herein incorporated by reference. The source of DNA or cDNA used will preferably have been enriched for HSF sequences. Such enrichment can most easily be obtained by using cDNA obtained by extracting RNA from cells cultured under conditions which induce HSF synthesis.

Techniques such as, or similar to, those described above have successfully enabled the cloning of genes for human aldehyde dehydrogenases (Hsu, L.C. et al., Proc. Natl. Acad. Sci. USA 82:3771-3775 (1985)), fibronectin (Suzuki, S. et al., Eur. Mol. Biol. Organ. J. 4:2519-2524 (1985)), the human estrogen receptor gene (Walter, P. et al., Proc. Natl. Acad. Sci. USA 82:7889-7893 (1985)), tissue-type plasminogen activator (Pennica, D. et al., Nature 301:214-221 (1983)) and human term placental alkaline phosphatase complementary DNA (Kam, W. et al., Proc. Natl. Acad. Sci. USA 82:8715-8719 (1985)).

In a preferred alternative way of cloning an HSF gene sequence, a library of expression vectors is prepared by cloning DNA or, more preferably cDNA, from a cell capable of expressing HSF, into an expression vector. The library is then screened for members capable of expressing a protein which binds to anti-HSF antibody, and which has a nucleotide sequence that is capable of encoding polypeptides that have the same amino acid sequence as HSF or fragments of HSF.

The cloned HSF gene, obtained through the methods described above, may be operably linked to an expression vector, and introduced into bacterial, or eukaryotic cells to produce HSF protein. Techniques for such manipulations are disclosed by Maniatis, T. et al., supra, and are well known in the art.

### Uses for HSF and its Functional Derivatives, Agonists and Antagonists

### A. Diagnostic Uses

The compounds of the present invention may be used to diagnose the presence of stress in organs or tissue, or in extracts, sections, etc. of organs or tissues. The compounds of the present invention can also be used to diagnose the level of stress in cells or cellular extracts. The ability to perform such a diagnosis is especially desirable in evaluating the suitability of organs (such as, for example, kidney, liver, heart, etc.) or tissue (skin, bone marrow, etc.) in organ or tissue transplant or replacement therapies.

The presence or level, of stress in a particular biological sample can be determined by identifying or quantifying the level of activated HSF which is present in that sample. Any of a variety of methods which are capable of identifying (or quantifying) the level of activated HSF in a sample can be used for this purpose. It is, however, most preferable to assay for activated HSF using an antibody, and especially a monoclonal antibody (or a fragment of either a polyclonal or a monoclonal antibody) which is capable of binding to activated HSF.

Diagnostic assays to detect activated HSF may comprise imaging assays (such as, for example, whole body or organ imaging) or may comprise a biopsy or in sito assay of cells or of organ or tissue sections. As indicated above, such assays may be conducted upon subcellular extracts from organs, tissues, cells, etc.

The antibodies (or fragments thereof) of the present invention are particularly suited for use in immunoassays wherein they may be utilized in liquid phase or bound to a solid-phase carrier.

Antibodies, or fragments thereof, may be labeled using any of a variety of labels and methods of labeling. Examples of types of labels which can be used in the present invention include, but are not limited to, enzyme labels, radioisotopic labels, non-radioactive isotopic labels, fluorescent labels, and chemiluminescent labels.

Examples of suitable enzyme labels include malate dehydrogenase, staphylococcal nuclease, delta-5-steroid isomerase, yeast-alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, acetylcholine esterase, etc.

Examples of suitable radioisotopic labels include ³H, ¹¹¹In, ¹²⁵I, ¹³¹I, ³²P, ³⁵S, ¹⁴C, ⁵¹Cr, ⁵⁷To, ⁵⁸Co, ⁵⁹Fe, ⁷⁵Se, ¹⁵²Eu, ⁹⁰Y, ⁶⁷Cu, ²¹⁷Ci, ²¹¹At, ²¹²Pb, ⁴⁷Sc, ¹⁰⁹Pd, etc. ¹¹¹In is a preferred isotope. Its use may have substantial advantages since its avoids the problem of dehalogenation of the ¹²⁵I or ¹³¹I-labeled monoclonal antibody by the liver. In addition, this radionucleotide has a more favorable gamma emission energy for imaging (Perkins, A.C., et al., Eur. J. Nucl. Med. 10:296-301 (1985); Carasquillo, J.A., et al., J. Nucl. Med. 28:281-287 (1987)).

Examples of suitable non-radioactive isotopic labels include ¹⁵⁷Gd, ⁵⁵Mn, ¹⁶²Dy, ⁵²Tr, ⁵⁶Fe, etc.

Examples of suitable fluorescent labels include a ¹⁵²Eu label, a fluorescein label, an isothiocyanate label, a rhodamine label, a phycoerythrin label, a phycocyanin label, an allophycocyanin label, an o-phthaldehyde label, a fluorescamine label, etc.

Examples of chemiluminescent labels include a luminal label, an isoluminal label, an aromatic acridinium ester label, an imidazole label, an acridinium salt label, an oxalate ester label, a luciferin label, a luciferase label, an aequorin label, etc.

Those of ordinary skill in the art will know of other suitable labels which may be employed in accordance with the present invention. The binding of these labels to antibodies or fragments thereof can be accomplished using standard techniques commonly known to those of ordinary skill in the art. Typical techniques are described by Kennedy, J.H., et al. (Clin. Chim. Acta 70:1-31 (1976)), and Schurs, A.H.W.M., et al. (Clin. Chim. Acta 81:1-40 (1977)). Coupling techniques mentioned in the latter are the glutaraldehyde method, the periodate method, the dimaleimide method, the m-maleimidobenzyl-N-hydroxy-succinimide ester method, all of which methods are incorporated by reference herein.

The detection of the antibodies (or fragments of antibodies) of the present invention can be improved through the use of carriers. Well-known carriers include glass, polystyrene, polypropylene, polyethylene, dextran, nylon, amylases, natural and modified celluloses, polyacrylamides, agaroses, and magnetite. The nature of the carrier can be either soluble to some extent or insoluble for the purposes of the present invention. The support material may have virtually any possible structural configuration so long as the coupled molecule is capable of binding to HSF. Thus, the support configuration may be spherical, as in a bead, or cylindrical, as in the inside surface of a test tube, or the external surface of a rod. Alternatively, the surface may be flat such as a sheet, test strip, etc. Those skilled in the art will note many other suitable carriers for binding monoclonal antibody, or will be able to ascertain the same by use of routine experimentation.

The antibodies, or fragments of antibodies, of the present invention may be used to quantitatively or qualitatively detect the presence of activated HSF. Such detection may be accomplished using any of a variety of immunoassays. For example, by radioactively labeling the antibodies or antibody fragments, it is possible to detect activated HSF through the use of radioimmune assays. Activated HSF can be distinguished from "preactivated" HSF by its capacity to bind to HSE-containing DNA. The amount of such activated HSF present in a sample can be measured using a radioimmune assay. A good description of a radioimmune assay (RIA) may be found in Laboratory Techniques and Biochemistry in Molecular Biology, by Work, T.S., et al., North Holland Publishing Company, NY (1978), with particular reference to the chapter entitled "An Introduction to Radioimmune Assay and Related Techniques" by Chard, T., incorporated by reference herein.

The antibody or antibody fragments of the present invention may also be adapted for utilization in an immunometric assay, also known as a "two-site" or "sandwich" assay. In a typical immunometric assay, a quantity of unlabeled antibody (or fragment of antibody) is bound to a solid support that is insoluble in the fluid being tested (i.e., blood, lymph, liquified stools, tissue homogenate, cellular extract etc.) and a quantity of detectably labeled soluble antibody is added to permit detection and/or quantitation of the ternary complex formed between solid-phase antibody, activated HSF, and labeled antibody.

Typical immunometric assays include "forward" assays in which the antibody bound to the solid phase is first contacted with the sample being tested to extract the activated HSF from the sample by formation of a binary solid phase antibody-HSF complex. After a suitable incubation period, the solid support is washed to remove the residue of the fluid sample, including unreacted antigen, if any, and then contacted with the solution containing an unknown quantity of labeled antibody (which functions as a "reporter molecule"). After a second incubation period to permit the labeled antibody to complex with the activated HSF bound to the solid support through the unlabeled antibody, the solid support is washed a second time to remove the unreacted labeled antibody. This type of forward sandwich assay may be a simple "yes/no" assay to determine whether activated HSF is present or may be made quantitative by comparing the measure of labeled antibody with that obtained for a standard sample containing known quantities of HSF. Such "two-site" or "sandwich" assays are described by Wide at pages 199-206 of Radioimmune Assay Method, edited by Kirkham and Hunter, E. & S. Livingstone, Edinburgh, 1970.

In another type of "sandwich" assay, which may also be useful to assay the activated HSF of the present invention, the so-called "simultaneous" and "reverse" assays are used. A simultaneous assay involves a single incubation step as the antibody bound to the solid support and labeled antibody are both added to the sample being tested at the same time. After the incubation is completed, the solid support is washed to remove the residue of fluid sample and uncomplex labeled antibody. The presence of labeled antibody associated with the solid support is then determined as it would be in a conventional "forward" sandwich assay.

In the "reverse" assay, stepwise addition first of a solution of labeled antibody to the fluid sample followed by the addition of unlabeled antibody bound to a solid support after a suitable incubation period is utilized. After a second incubation, the solid phase is washed in conventional fashion to free it of the residue of the sample being tested and the solution of unreacted labeled antibody. The determination of labeled antibody associated with a solid support is then determined as in the "simultaneous" and "forward" assays.

As explained above, the immunometric assays for HSF require that the particular binding molecule be labeled with a "reporter molecule." These reporter molecules or labels, as identified above, are conventional and well-known to the art. In the practice of the present invention, enzyme labels are a preferred embodiment. No single enzyme is ideal for use as a label in every conceivable immunometric assay. Instead, one must determine which enzyme is suitable for a particular assay system. Criteria important for the choice of enzymes are turnover number of the pure enzyme (the number of substrate molecules converted to product per enzyme site per unit of time), purity of the enzyme preparation, sensitivity of detection of its product, ease and speed of detection of the enzyme reaction, absence of interfering factors or of enzyme-like activity in the test fluid, stability of the enzyme and its conjugate, availability and cost of the enzyme and its conjugate, and the like. Included among the enzymes used as preferred labels in the immunometric assays of the present invention are peroxidase, alkaline phosphatase, beta-galactosidase, urease, glucose oxidase, glycoamylase, malate dehydrogenase, and glucose-6-phosphate dehydrogenase. Urease is among the more preferred enzyme labels, particularly because of chromogenic pH indicators which make its activity readily visible to the naked eye.

### B. Therapeutic Uses

Agents which increase the level of activated HSF in a subject (i.e. a human or an animal) may be used in the therapy of any disease associated with the heat shock stress response. As discussed above, the heat shock response is a very highly evolutionarily conserved pathway that an organism uses to respond to a variety of stresses. Included in these stresses are hypoxia and ethanol.

The pathophysiologic manifestations of hypoxia are well documented. Hypoxia is the mechanism that is directly responsible for both myocardial and cerebral infarctions. Hence, if a human or animal were better able to respond to an hypoxic insult, it would potentially survive the insult with less permanent damage. The limitation of myocardial infarct size has been shown to directly affect patient survival. Agents which increase the level of activated HSF in a subject thus have utility in potentiating such stress. Such therapy is also valuable in the treatment of ethanol induced stress, due to its capacity to limit the metabolic derangement that results from an overdose of ethanol.

In providing a patient with antibodies, or fragments thereof, capable of binding to HSF, or when providing HSF (or a fragment, variant, or derivative thereof) or an agent capable of promoting the activation of HSF to a recipient patient, the dosage of administered agent will vary depending upon such factors as the patient's age, weight, height, sex, general medical condition, previous medical history, etc. In general, it is desirable to provide the recipient with a dosage of antibody which is in the range of from 1 pg/kg to 10 mg/kg (body weight of patient), although a lower or higher dosage may be administered. When providing the above-described compounds to a patient, it is preferable to administer such compounds in a dosage which also ranges from 1 pg/kg to 10 mg/kg (body weight of pateint) although a lower or higher dosage may also be administered.

The compounds of the present invention may be administered to patients intravenously, intramuscularly, subcutaneously, enterally, or parenterally. When administering such compounds by injection, the administration may be by continuous infusion, or by single or multiple boluses.

The compounds of the present invention are intended to be provided to recipient subjects in an amount sufficient to effect the heat shock response. An amount is said to be sufficient to "effect" the heat shock response if the dosage, route of administration, etc. of the agent are sufficient to influence such a response.

The compounds of the present invention may be provided either prior to the onset of a stress-causing condition (so as to suppress the anticipated damage of such a condition) or after the initiation of the condition.

A composition is said to be "pharmacologically acceptable" if its administration can be tolerated by a recipient patient. Such an agent is said to be administered in a "therapeutically effective amount" if the amount administered is physiologically significant. An agent is physiologically significant if its presence results in a detectable change in the physiology of a recipient patient.

The compounds of the present invention can be formulated according to known methods to prepare pharmaceutically useful compositions, whereby these materials, or their functional derivatives, are combined in admixture with a pharmaceutically acceptable carrier vehicle. Suitable vehicles and their formulation, inclusive of other human proteins, e.g., human serum albumin, are described, for example, in Remington's Pharmaceutical Sciences (16th ed., Osol, A., Ed., Mack, Easton PA (1980)). In order to form a pharmaceutically acceptable composition suitable for effective administration, such compositions will contain an effective amount of the above-described compounds together with a suitable amount of carrier vehicle.

Additional pharmaceutical methods may be employed to control the duration of action. Control release preparations may be achieved through the use of polymers to complex or absorb the compounds. The controlled delivery may be exercised by selecting appropriate macromolecules (for example polyesters, polyamino acids, polyvinyl, pyrrolidone, ethylenevinylacetate, methylcellulose, carboxymethylcellulose, or protamine, sulfate) and the concentration of macromolecules as well as the methods of incorporation in order to control release. Another possible method to control the duration of action by controlled release preparations is to incorporate the compounds of the present invention into particles of a polymeric material such as polyesters, polyamino acids, hydrogels, poly(lactic acid) or ethylene vinylacetate copolymers. Alternatively, instead of incorporating these agents into polymeric particles, it is possible to entrap these materials in microcapsules prepared, for example, by coacervation techniques or by interfacial polymerization, for example, hydroxymethylcellulose or gelatine-microcapsules and poly(methylmethacylate) microcapsules, respectively, or in colloidal drug delivery systems, for example, liposomes, albumin microspheres, microemulsions, nanoparticles, and nanocapsules or in macroemulsions. Such techniques are disclosed in Remington's Pharmaceutical Sciences (1980).

Having now generally described the invention, the same will be more readily understood through reference to the following examples which are provided by way of illustration, and are not intended to be limiting of the present invention, unless specified.

### EXAMPLE 1

### ASSAY FOR HEAT SHOCK FACTOR

Human HSF bound to the HSE can be detected as a complex that migrates with a characteristic mobility on a non-denaturing polyacrylamide gel (Kingston R.E. et al., Mol. Cell. Biol. 7:1530 (1987); Sorger, P.K. et al., Nature 329:81 (1987), Nuclear extracts from HeLa cells growing at 43°C contain significantly more of this binding activity than do nuclear extracts from HeLa cells growing at 37°C In order to develop an in vitro system to study this induction, nuclear and cytoplasmic extracts were prepared from HeLa cells growing at 37°C (Dignam, D. et al., Nucl. Acids Res 11:1475 (1983), which reference is hereby incorporated by reference herein). These extracts were heated at 43°C for one hour and the level of HSE binding activity was determined using the band retention technique.

Nuclear and cytoplasmic extracts from 4 L of HeLa cells growing at 37°C (denoted NE and S100, respectively), and nuclear extracts from 1 L of HeLa cells immediately after a 1 hr incubation at 43°C (HSNE) were prepared as described by Dignam et al. (Nucl. Acids Res 11:1475 (1983)). Extracts were dialyzed into buffer (20 mM Hepes, pH 7.9, 20% (vol/vol) glycerol, 100 mM KCl, 0.2 mM EDTA, 0.5 mM phenylmethylsulfonyl fluoride, 0.5 mM dithiothreitol) and were incubated without any addition. Protein (5.0 »g of protein of the NE or 8.5 »g S100, or both) were incubated for 1 hr at either 0°C or 43°C. The extracts were then incubated at 30°C for 30 min with a ³²P-labeled synthetic oligonucleotide containing the HSE (bases -11 to-80) of the human hsp70 promoter (Sen, R. et al.; Cell 47:921 (1986); Prywes, R. et al., Cell 47:777 (1986); Hayes, T.E. et al., Proc. Natl. Acad. Sci. USA 84:1272 (1987)). Binding reaction mixtures (20 »l) contained 12 mM HEPES, pH 7.9, 12% (vol/vol) glycerol, 60 mM KCl, 0.12 mM EDTA, 0.3 mM phenylmethylsulfonyl fluoride, 0.3 mM dithiothreitol, 2 mM MgCl₂, 100 »g of poly(dI-dC)·poly(dI-dC) per mol, approximately 0.2 ng of the labeled DNA, and extract. The reaction mixtures were then subjected to electrophoresis on a 4% nondenaturing polyacrylamide gel.

A 36-mer double stranded oligonucleotide containing the HSE (bases -80 to -115 of the human hsp70 promoter) was used as labelled probe. The nuclear extract contained no binding activity either before or after heating, while the cytoplasmic extract was found to have significant levels of binding activity only after incubation at 43°C. Addition of nuclear extract to cytoplasmic extract did not alter the extent of induction.

The results of this experiment show that cytoplasmic extracts from non-heat induced HeLa cells contain a heat inducible activity binding in the region of the heat shock element determined by a gel mobility shift assay.

### EXAMPLE 2

### PURIFICATION OF THE HEAT SHOCK FACTOR

### A) Preparation of Nuclear Extract

Human HeLa cells were grown in suspension culture supported by Minimal Essential Media (Joklik's Modification) which was supplemented with 5% Horse serum. Cells were grown to a density of approximately 500,000 cells per milliliter. Cells were then harvested and nuclear extracts were prepared essentially as described by Dignam et al. (Nucl. Acids Res 11:1475 (1983). The nuclear extract was dialyzed into Dignam Buffer D (20% v/v glycerol, 20mM HEPES (pH 7.9), 0.1M KCl, 0.5mM DTT, 0.5mM PMSF, 0.2mM EDTA).

### B) Fractionation Step #1

The HeLa cell nuclear extract in Dignam Buffer D was passed over a Calf Thymus Double Stranded DNA-Cellulose Column (Pharmacia) which had been pre-equilibrated in Buffer D. The column was eluted with step increments of Buffer D containing 0.3M KCl and 1.5M KCl. The HSF activity was monitored with the gel electrophoresis mobility shift assay of Kingston et al. (Mol. Cell. Biol. 7:1530 (1987)). The HSF activity was found to elute at 0.3M KCl. Fractions containing HSF activity were pooled and the detergent NP-40 was added to 0.1% (v/v).

### C) Fractionation Step #2

The second purification step relied on the specific binding of HSF to its cognate sequence -- the HSE. For this step, a sequence-specific double-stranded oligonucleotide affinity column was constructed according to the method of Kadonaga et al. (Proc. Natl. Acad. Sci. USA 83:5889 (1986)). A self-complimentary 28 base oligonucleotide was synthesized with the following sequence:
5′- GATCCTAGAAGCTTCTAGAAGCTTCTAG - 3′
This oligonucleotide was self-annealed to form a double-stranded molecule with GATC 5′ overhangs and two overlapping HSEs. These molecules were phosphorylated and ligated into concatamers with an average length of about 5 to 10 units. This DNA was covalently attached to Sepharose® CL-2B (Pharmacia) using cyanogen bromide. This, then, constituted the affinity column.

Pooled fractions from Step #1 were applied to the affinity column which had been pre-equilibrated in Buffer 0.3D′ins (Buffer D with 0.3M KCl, 0.1% v/v NP-40, and 0.1 mg/ml bovine insulin). The HSF activity was eluted with one step wash of Buffer 0.8D′ins (0.8M KCl, 0.1% v/v NP-40, and 0.1 mg/ml bovine insulin).

### D) Fractionation Step #3

Pooled fractions from the previous step were diluted to 0.1M KCl and were applied to MONO Q FPLC column (Pharmacia). The column was eluted with a linear gradient of KCl from 0.1 to 1.0M fractions KCL in Buffer D′ins. HSF activity was assayed and active fractions were pooled. The eluted material produced 2 bands when analyzed by SDS gel electrophoresis. One of these bands is thought to be a degradation product of the other. Thus, the material is between 50 % pure (if the two bands are unrelated) and 95 % pure (if the two bands are related).

### EXAMPLE 3

### CHARACTERIZATION OF THE HEAT SHOCK FACTOR

The cytoplasmic heat-inducible binding activity formed a single retained band that comigrated with the lower of two retained bands obtained with nuclear extract from heat shocked cells. Fractionation of the cytoplasmic extract on either phosphocellulose or Biorex 70 yields a fraction that produces two bands of appropriate mobility after incubation at 43°C. The presence of only the lower band after induction of the unfractionated extract at 43°C may therefore be due to an inhibitor in the extract. The sequence specificity of this binding was examined in order to verify that the binding represented an interaction with the HSE.

The sequence specificity of the heat-inducible binding activity present in the S100 extract was characterized by examining the competition of the heat-induced band with fragments containing the heat shock element and nonspecific fragments. Competition of the heat-induced band with fragments containing the heat shock element and nonspecific fragments was investigated as follows. 8.5 »g of the S100 protein was incubated (or not incubated) at 43°C for 1 hr prior to incubation with the DNA probe. S100 extracts were prepared and DNA incubations and electrophoresis were performed as described above. Incubation mixtures also contained either nonradioactive competitor DNA (bases -84 to +5 of the human hsp70 gene (which does not contain the heat shock element) in a 5- or 10-fold molar excess or bases -148 to -74 of the human hsp70 gene (which contains the heat shock element centered at base -100) or a 310-base-pair fragment of a D. melanogaster heat shock gene containing three heat shock elements (XhoI [base -200] to EcoRI [base +110], of plasmid pSP6-HS-9 (Wurm, F.M. et al., Proc. Natl. Acad. Sci. USA 83:5415 (1986)), derived from cloned fragment 232 described by Holmgren et al. (Cell 18:1359 (1979) or a 39-base-pair double-stranded synthetic oligonucleotide containing the CAAT sequence (top strand, 5′-CA CCG TCG ATT TCC CTT CTG AGC CAA TCA CCG AGC TCG A); or the 36-base-pair double-stranded synthetic oligonucleotide probe. DNA concentrations were estimated by agarose gel electrophoresis.

To further characterize the binding specificity, a methylation interference analysis was performed (Siebenlist, U. et al., Proc. Natl. Acad. Sci. USA 77:122 (9180); Gilman, M.Z. et al., Mol. Cell. Biol. 6:4305 (1986)). A partially methylated fragment containing the HSE was incubated with heated cytoplasmic extract, and bound and free fragments were separated and analyzed. The analysis was performed with radioactive label on either the top or bottom strand. Cytoplasmic extract was heated at 43°C for 1.5 hr. Binding reactions were performed as described above In such assays, 120 »g of heated cytoplasmic extract (IN VITRO) or 16 »g HSNE (IN VIVO) were incubated with 20 ng of a labeled fragment containing bases -148 to -74 of the promoter. Bound and free complexes were separated on a nondenaturing gel, eluted, and analyzed as described by Gilman, M.Z. et al. (Mol. Cell. Biol. 6:4305 (1986)). The heat shock consensus sequence is C--GAA--TTC--G. All fragments that contained in HSE competed for binding, while there was no competition by fragments that did not contain the HSE. Five G residues in or adjacent to the HSE were underrepresented in bound DNA, indicating that the bound factor is in close contact with these residues. A similar pattern is seen with the factor found in nuclear extracts from heat-shocked HeLa cells. The competition data and this DMS interference data show that the binding activity induced in vitro has the same sequence specificity as that seen after induction in whole cells (Kingston R.E. et al., Mol. Cell. Biol. 7:1530 (1987)).

### EXAMPLE 4

### CHARACTERIZATION OF THE INDUCTION OF BINDING

The induction of binding activity in intact HeLa cells was compared with the induction of HSE in a cell-free system. The induction of binding activity in intact HeLa cells was found to occur with rapid kinetics, whereas induction in the cell-free system occurs more slowly. Determinations of the kinetics and temperature dependence of the heat-inducible binding activity present in the S100 extract were performed as follows. S100 extracts were prepared and DNA incubation and electrophoresis were performed as described in the above. Samples contained 8.5 »g of protein from the S100 extract. Samples were incubated for 1 hr. at 43°C prior to performing the binding reactions. After this incubation, some samples were then incubated for 1 additional hr. at 37°C, 30°C, or 0°C prior to binding reactions. S100 extracts were incubated at 43°C prior to binding reactions. Peak levels of binding activity were seen after 45 to 60 minutes of incubation at 43°C. The optimal temperature for induction of binding activity closely correlated with the temperature at which HeLa cells undergo the heat shock response. There was a very slight induction of the binding ability at 37°C in vitro, with increased induction seen at 40°C and 43°C and no induction was seen at 50°C. Once binding ability was induced, it was found to be stable; after induction at 43°C, further incubation on ice, at 30°C or at 37°C for up to one hour did not result in deactivation of the activated factor.

### EXAMPLE 5

### INDUCTION OF HSF FROM HELA CELLS

The amount of HSF in a cytoplasmic extract that had been heated for one hour at 43°C was determined by using the mobility shift gel under conditions where the amount of measured HSF increased linearly with increasing protein. The amount of HSF in a nuclear extract from HeLa cells heated intact at 43°C was measured in the same manner. The activities were then corrected for the number of cells that had been used to produce the respective extracts. The amount of HSF in the heated cytoplasmic extract was 12,200 units per 10⁶ cells, while the nuclear extract from heated cells contained 11,800 units per 10⁶ cells. Units are defined by the amount of labelled HSE probe retained on a mobility shift gel.

The characteristics of the in vitro induction of binding ability of HSF strongly suggest that this activation mirrors that seen when intact HeLa cells are heat-shocked. HSE-binding specificity after in vitro activation is identical to that seen when factor is isolated from heat-shocked HeLa cells. The temperature profile for induction in vitro is similar to that seen in intact cells. Additionally, activation of HSe-binding activity in vitro is efficient. Induction of a cytoplasmic extract in vitro results in 12.2 units of HSF per 10⁶ cells, while nuclei of heat-shocked HeLa cells contain 11.8 units of HSF per 10⁶ cells.

### EXAMPLE 6

### EFFECT OF TEMPERATURE ON HSF ACTIVATION

Heated cells contain increased levels of denatured protein, and it has been suggested that this triggers activation of HSF via a protein degradation pathway (Anantham, J. et al., Science 232:522 (1986); Finley, D. et al., Cell 17:43 (1984); Goff, S.A. et al., Cell 41:587 (1985); Munro, S. et al., Nature (London) 317:477 (1985)). This model predicts that activation occurs in a temperature dependent fashion in the in vitro system because there is more denatured protein at 43°C than at 37°C. In contrast, addition of boiled BSA to the cytoplasmic extract did not change the rate of induction of HSF at either 37°C or 43°C (see above) nor did it change the extent of induction. Addition of boiled cytoplasmic extract also had very little effect on activation. Thus, denatured protein does not play a role in changing HSF to a form that can bind DNA, suggesting that this alteration may result from a direct effect of temperature.

### EXAMPLE 7

### MOLECULAR MECHANISM OF HSF ACTIVATION

Fractionation of the cytoplasmic extract on several ion-exchange columns results in individual fractions that retain the ability to activate HSF in a heat-inducible manner. These fractions were used to show that addition of ATP has no effect on the rate or extent of activation of binding activity. A cytoplasmic extract was applied to a Biorex 70 column an diluted with washes containing 0.1, 0.2, 0.5 and 1.0 M KCl. Fractions were assayed individually and in combination for the ability to form active HSF upon incubation at 43°C. All of this activity eluted in the 0.1 M KCl wash. One of the protein factors retained on this column (" X ") was found to have a faster mobility than HSF. The activity that form X eluted in the 0.5 M KCl wash. The 0.5 M wash had no ability to form active HSF, and did not alter the amount of HSF formed when added to the 0.1 M KCl wash. This finding is consistent with a model where an individual, inactive HSF molecule undergoes a heat-induced conformation change to a DNA-binding form. However, the extent of activation of HSF is exquisitely sensitive to dilution of the cytoplasmic extract, indicating that at least two molecules are needed to activate HSF. These results indicate either that (1) inactive HSF exists as a monomer which undergoes a conformational change and dimerization to form an active species capable of binding, or (2) a second, heat-sensitive factor exists which modifies HSF to a DNA-binding form.

As indicated above, the assay of HSF activation revealed the presence of a band (" X ") whose level was inversely correlated with the amount of HSF. This finding suggests that this band represents a precursor of active HSF. However, the activity that forms this band separates from the activity that forms active HSF on negatively charged columns. Thus, it is unlikely that the factor that produces X is a precursor to active HSF, although a role for X in regulation of the heat shock response remains possible.

### EXAMPLE 8

### CROSSLINKING STUDIES

The characteristics of activation of HSF in vitro were investigated by assessing the level of HSF present after the following manipulations as determined by the above described gel mobility shift assay using a synthetic oligonucleotide containing an HSE (see above).
A) 20 »g of S100 protein was incubated for 20 min in the presence or absence of boiled bovine serum albumin (10 »g BSA protein/20 »g S100 protein). This experiment revealed that the addition of denatured protein does not alter activation.
B) Cytoplasmic extract was diluted with an equal volume, 4 vol or 9 vol of boiled cytoplasmic extract prior to incubation for 90 min. Activity was then assayed in binding reactions (20 »l) that all contained 13 »g of active (nonboiled) cytoplasmic extract. This experiment revealed that dilution reduced activation of HSF in vitro. Similar results were obtained after dilution with buffer.
C) Cytoplasmic extract (13 mg/ml) was either heated for 1 hr. at 43°C ("43°C extract") or incubated on ice for 1 hr. ("0°C extract"). The 43°C extract (either 1 »l, 3 »l, or 0 »l) was incubated with an amount of 0°C extract (either 0 »l, 2 »l or 4 »l) at 30°C for 1 hr. This experiment revealed that addition of non-heated extract did not inhibit binding to the HSE.

The HSF band had the following characteristics: it is not present in control nuclear extracts; it is present in the HSF-HSE complex identified on mobility shift gels; and its formation is efficiently competed by an oligonucleotide that contains an HSE, but not by a control oligonucleotide specifying the CCAAT element.

UV crosslinking studies were performed in order to determine the apparent size of each factor. Extracts were incubated with a synthetic, dimerized HSE that had been substituted with α ³²P-CTP and bromo-deoxyuridine, and the resultant HSF containing complex was separated from non-specific complexes on an agarose gel and irradiated with UV light. Analysis of the resultant labelled protein species on an SDS polyacrylamide gel revealed a band migrating at 93 kd that was present in nuclear extracts from heated HeLa cells.

Surprisingly, similar experiments with the heated cytoplasmic extract revealed that HSF activated in vitro runs markedly faster (90 kd) on a polyacrylamide gel than HSF isolated from nuclei of heated cells. It is not present in cytoplasmic extract that has not been heated, and formation of the crosslinked complex is competed by an HSE but not by a control CCAAT oligonucleotide. Thus, HSF activated for DNA-binding specificity in vitro differs in some way from HSF isolated from heat shocked cells. This difference is not caused by an activity in cytoplasmic extracts that modifies active HSF, as both the 93 kd and 90 kd form of HSF are observed after heating a cytoplasmic extract in the presence of nuclear extract.

One possibility for this difference in mobility, by analogy with Yeast HSF, is phosphorylation. Yeast HSF increases in apparent molecular weight upon heat shock of yeast cells, and this change appears to be caused by phosphorylation. HSF from heated HeLa cells and in vitro activated HSF were treated with acid phosphatase prior to crosslinking the factor to DNA. Treatment with acid phosphatase increased the apparent mobility of HSF that had been isolated from heated HeLa cells, while treatment with calf intestinal phosphatase had a limited effect. Treatment of HSF from heated cells with both phosphatases resulted in a diffuse band that extended from approximately 90 kd to 92 kd. In contrast, there was no detectable effect of acid phosphatase on the mobility of the in vitro activated HSF. Although phosphatase treatment did not change the mobility of HSF from heated cells to precisely that of in vitro activated HSF, these results imply that HSF found in intact heated cells is more extensively phosphorylated than HSF that has been activated in vitro.

The HSE-binding capabilities of human HSF can, thus, be activated by heating a HeLa cytoplasmic extract. Denatured protein does not alter the rate or extent of activation of the HSE binding activity in vitro. HSF that has been activated in vitro has DNA-binding capabilities that are identical to HSF isolated from the nuclei of heated human cells, however the in vitro activated HSF runs with faster mobility on polyacrylamide gels than does HSF activated in intact cells. This difference in mobility appears to be due to differences in phosphorylation. Thus, human cells respond to heat by activating HSF by at least two steps: first, an ATP-independent heat-induced alteration in DNA-binding capability, followed by phosphorylation.

## Claims (Claims for the following Contracting State(s): AT, BE, CH, DE, FR, GB, IT, LI, LU, NL, SE)

1. Human Heat Shock Factor (hHSF) obtainable by
(a) preparing nuclear extracts;
(b) applying said extracts of step (a) to a double standed DNA-Cellulose Column;
(c) applying the active fractions of step (b) to a sequence-specific double-standed oligonucleotide affinity column; and
(d) applying the active fractions of step (c) to a MONO Q FPLC column.

2. hHSF of claim 1 wherein said hHSF is human HELA Heat Shock Factor or a derivative of human HELA Heat Shock Factor.

3. A therapeutic medicament comprising isolated hHSF according to claim 1, or derivatives thereof.

4. A recombinant nucleic acid molecule comprising a sequence which encodes the hHSF of claim 1 or 2.

5. Immunoglobulins acting as antagonists of a Heat Shock Factor.

6. A method for purifying hHSF which comprises
(a) preparing nuclear extracts;
(b) applying said extracts of step (a) to a double standed DNA-Cellulose Column;
(c) applying the active fractions of step (b) to a sequence-specific double-standed oligonucleotide affinity column; and
(d) applying the active fractions of step (c) to a MONO Q FPLC column.

7. A method for diagnosing the stress condition of a patient which comprises assaying, preferably in a biopsy, for the presence of activated Heat Shock Factor in a cell of an individual.

8. The method of claim 7 wherein said assay comprises the use of an antagonist of Heat Shock Factor.

9. The method of claim 8 wherein said antagonist is an antibody, preferably a monoclonal antibody, or a fragment of an antibody.

## Claims (Claims for the following Contracting State(s): ES)

1. A method for purifying human HEAT Shock Factor which comprises
(a) preparing nuclear exracts;
(b) applying said extracts of step (a) to a double standed DNA-Cellulose Column;
(c) applying the active fractions of step (b) to a sequence-specific double-standed oligonucleotide affinity column; and
(d) applying the active fractions of step (c) to a MONO Q FPLC column.

## Claims (Claims for the following Contracting State(s): GR)

1. A method for purifying human HEAT Shock Factor (hHSF) which comprises
(a) preparing nuclear extracts;
(b) applying said extracts of step (a) to a double standed DNA-Cellulose Column;
(c) applying the active fractions of step (b) to a sequence-specific double-standed oligonucleotide affinity column; and
(d) applying the active fractions of step (c) to a MONO Q FPLC column.

2. A recombinant nucleic acid molecule comprising a sequence which encodes hHSF or a derivative thereof.

## Patentansprüche (Patentansprüche für folgende(n) Vertragsstaat(en): AT, BE, CH, DE, FR, GB, IT, LI, LU, NL, SE)

1. Human-Hitzeschock-Faktor (hHSF), erhältlich durch:
(a) Herstellen von Zellkernextrakten;
(b) Aufgeben dieser Extrakte aus der Stufe (a) auf eine Zellulose-Säule mit doppelsträngiger DNA;
(c) Aufgeben der aktiven Fraktionen aus der Stufe (b) auf eine Affinitätschromatographie-Säule mit einem sequenzspezifischen, doppelsträngigen Oligonukleotid; und
(d) Aufgeben der aktiven Fraktionen aus der Stufe (c) auf eine MONO Q FPLC-Säule.

2. hHSF nach Anspruch 1, wobei dieser hHSF ein Human-HELA-Hitzeschock-Faktor oder ein Derivat des Human-HELA-Hitzeschock-Faktors ist.

3. Therapeutisches Medikament, welches den isolierten hHSF nach Anspruch 1 oder Derivate hievon enthält.

4. Rekombinantes Nukleinsäuremolekül, welches eine Sequenz enthält, welche für den hHSF nach Anspruch 1 oder 2 kodiert.

5. Immunglobuline, welche als Antagonisten eines Hitzeschock-Faktors wirken.

6. Verfahren zum Isolieren und Reinigen von hHSF, welches umfaßt:
(a) Herstellen von Zellkernextrakten;
(b) Aufgeben dieser Extrakte aus der Stufe (a) auf eine Zellulose-Säule mit doppelsträngiger DNA;
(c) Aufgeben der aktiven Fraktionen aus der Stufe (b) auf eine Affinitätschromatographie-Säule mit einem seguenzspezifischen, doppelsträngigen Oligonukleotid; und
(d) Aufgeben der aktiven Fraktionen aus der Stufe (c) auf eine MONO Q FPLC-Säule.

7. Verfahren zum Diagnostizieren des Streßzustandes eines Patienten, welches das Prüfen, vorzugsweise in einer Biopsie, auf das Vorliegen des aktivierten Hitzeschock-Faktors in einer Zelle eines Individuums umfaßt.

8. Verfahren nach Anspruch 7, wobei der Test die Verwendung eines Antagonisten des Hitzeschock-Faktors umfaßt.

9. Verfahren nach Anspruch 8, wobei der Antagonist ein Antikörper, vorzugsweise ein monoklonaler Antikörper, oder ein Fragment eines Antikörpers ist.

## Patentansprüche (Patentansprüche für folgende(n) Vertragsstaat(en): ES)

1. Verfahren zum Isolieren und Reinigen von Human-Hitzeschock-Faktor, welches umfaßt:
(a) Herstellen von Zellkernextrakten;
(b) Aufgeben dieser Extrakte aus der Stufe (a) auf eine Zellulose-Säule mit doppelsträngiger DNA;
(c) Aufgeben der aktiven Fraktionen aus der Stufe (b) auf eine Affinitätschromatographie-Säule mit einem sequenzspezifischen, doppelsträngigen Oligonukleotid; und
(d) Aufgeben der aktiven Fraktionen aus der Stufe (c) auf eine MONO Q FPLC-Säule.

## Patentansprüche (Patentansprüche für folgende(n) Vertragsstaat(en): GR)

1. Verfahren zum Isolieren und Reinigen von Human-Hitzeschock-Faitor (hHSF), welches umfaßt:
(a) Herstellen von Zellkernextrakten;
(b) Aufgeben dieser Extrakte aus der Stufe (a) auf eine Zellulose-Säule mit doppelsträngiger DNA;
(c) Aufgeben der aktiven Fraktionen aus der Stufe (b) auf eine Affinitätschromatographie-Säule mit einem sequenzspezifischen, doppelsträngigen Oligonukleotid; und
(d) Aufgeben der aktiven Fraktionen aus der Stufe (c) auf eine MONO Q FPLC-Säule.

2. Rekombinantes Nukleinsäuremolekül, welches eine Sequenz enthält, welche für den hHSF oder ein Derivat hievon kodiert.

## Revendications (Revendications pour l'(les) Etat(s) contractant(s) suivant(s): AT, BE, CH, DE, FR, GB, IT, LI, LU, NL, SE)

1. Facteur du choc thermique humain (hHSF) pouvant être obtenu par
(a) préparation d'extraits nucléaires;
(b) injection desdits extraits de l'étape (a) dans une colonne de cellulose-ADN bicaténaire;
(c) injection des fractions actives de l'étape (b) dans une colonne d'affinité-oligonucléotide bicaténaire spécifique de séquence; et
(d) injection des fractions actives de l'étape (c) dans une colonne de FPLC MONO Q.

2. hHSF de la revendication 1, caractérisé en ce que ledit hHSF est un facteur du choc thermique HeLa humain ou un dérivé du facteur du choc thermique HeLa humain.

3. Médicament thérapeutique comprenant du hHSF isolé selon la revendication 1, ou des dérivés de celui-ci.

4. Molécule d'acide nucléique recombinant, comprenant une séquence qui code pour le hHSF de la revendication 1 ou 2.

5. Immunoglobulines agissant en tant qu'antagonistes d'un facteur du choc thermique.

6. Procédé pour la purification du hHSF, comprenant
(a) la préparation d'extraits nucléaires;
(b) l'injection desdits extraits de l'étape (a) dans une colonne de cellulose-ADN bicaténaire;
(c) l'injection des fractions actives de l'étape (b) dans une colonne d'affinité-oligonucléotide bicaténaire spécifique de séquence; et
(d) l'injection des fractions actives de l'étape (c) dans une colonne de FPLC MONO Q.

7. Procédé pour le diagnostic de l'état de stress d'un patient, comprenant un essai, de préférence dans une biopsie, pour la détermination de la présence de facteur activé du choc thermique dans une cellule d'un individu.

8. Procédé de la revendication 7, dans lequel ledit essai comprend l'utilisation d'un antagoniste du facteur du choc thermique.

9. Procédé de la revendication 6, dans lequel ledit antagoniste est un anticorps, de préférence un anticorps monoclonal, ou un fragment d'un anticorps.

## Revendications (Revendications pour l'(les) Etat(s) contractant(s) suivant(s): ES)

1. Procédé pour la purification du facteur du choc thermique humain, comprenant
(a) la préparation d'extraits nucléaires;
(b) l'injection desdits extraits de l'étape (a) dans une colonne de cellulose-ADN bicaténaire;
(c) l'injection des fractions actives de l'étape (b) dans une colonne d'affinité-oligonucléotide bicaténaire spécifique de séquence; et
(d) l'injection des fractions actives de l'étape (c) dans une colonne de FPLC MONO Q.

## Revendications (Revendications pour l'(les) Etat(s) contractant(s) suivant(s): GR)

1. Procédé pour la purification du facteur du choc thermique humain (hHSF), comprenant
(a) la préparation d'extraits nucléaires;
(b) l'injection desdits extraits de l'étape (a) dans une colonne de cellulose-ADN bicaténaire;
(c) l'injection des fractions actives de l'étape (b) dans une colonne d'affinité-oligonucléotide bicaténaire spécifique de séquence; et
(d) l'injection des fractions actives de l'étape (c) dans une colonne de FPLC MONO Q.

2. Molécule d'acide nucléique recombinant, comprenant une séquence qui code pour le hHSF ou un dérivé de celui-ci.
